# EUROPEAN PATENT SPECIFICATION

(11) **EP 2 108 344 B1**
(45) Date of publication and mention of the grant of the patent: **26.10.2011**
(21) Application number: 08075292.6
(22) Date of filing: 11.04.2008
(51) Int. Cl.: A61F 5/44, A61F 5/445

(54) **An integral soft and flexible tail closure for a drainable stoma pouch with finger pocket**
Integrierter weicher und biegsamer Klammerverschluss für einen ablassbaren Stomabeutel mit Fingertasche
Fermeture d'extrémité flexible et souple intégrale pour poche de stomie vidable avec une poche d'extraction

(43) Date of publication of application: 14.10.2009
(73) Proprietor: Eurotec Beheer B.V., 4705 AG Roosendaal (NL)
(72) Inventor: Van der Leden, Arie Gijsbert, 2920 Kalmthout (BE)
(74) Representative: Groot Koerkamp, Jasper Henri

(56) References cited:
- WO-A-2006/031275
- DE-A1- 19 724 079
- GB-A- 1 370 622
- US-A1- 2008 033 379

## Description

### Background of the invention

A number of diseases involving the intestinal and urinary tracts may result in the construction of an artificial outlet on the abdominal area for stool and/or urine.

In the case of diversion of the large intestine into the abdominal wall, one talks about a colostomy. In the case of diversion of the small intestine one talks about an ileostomy. Thirdly in the case of an urostomy, the urine is disposed through an artificial opening in the abdominal wall. The artificial openings of a colostomy, ileostomy and a urostomy are collectively referred to as stomas. During the past years a large variety of stoma appliances have been developed. Patients with a colostomy collect their relatively solid faeces in closed pouches, which usually are changed several times a day. Patients with an ileostomy collect their relatively liquid stool in drainable pouches, the opening of which can be opened and closed with a tail clip. Patients with a urostomy collect their urine in a pouch with a drain tap. Ileostomy and urostomy pouches are usually changed every day.

Stoma appliances are to be divided in two main **systems; the 1-piece and the 2-piece system.** The design of most 2-piece stoma systems is based on the 2-piece stoma system designed by Steer et al. (British patent application 571, 657). This stoma system consists of a body side wafer produced from a special hydrocolloid adhesive, covered by a thin polyethylene film, on which a circular shaped polymeric flange with an upstanding or projecting rib has been attached, which functions as a coupling member. The stoma pouch is also provided with a circular shaped coupling member, which can be attached on or around the projecting rib of the body side flange.

US 2008/003379 discloses a drainable stoma pouch having a discharge opening that is closed by folding the neck portion upwardly and opened by unfolding the neck portion downwardly. Each of the sidewalls of the neck portion is provided with a transversely-extending bias member. The bias members are openable under inwardly-directed finger pressure applied to their opposite ends.

In the case of a 1-piece system, the skin barrier - which consists of a special adhesive - is integral with the pouch. This type of pouch is designed to be affixed directly to the skin, and has the advantage that the total surface of the adhesive is flatter and more flexible, which allows it more or less to follow the contours of the skin.

People with an ileostomy use to have a constant flow of liquid stool, to be collected in drainable pouches, at choice from a 1-piece or a 2-piece system, which pouches need to be emptied several times a day. For that purpose these stoma pouches are provided with a bottom tail, which can be opened and closed with a separate tail clip. These types of rigid plastic tail clips are available in a wide variety and offered by the manufacturers of said drainable pouches. The past decade, a lot of alternative integral stoma pouch locking systems were developed, sometimes new, sometimes based upon older inventions. These integral pouch locking systems use to consist of two strips on the bottom of the pouch, with which the tail of the pouch can be rolled up and locked with a velcro type 'loop and hook' locking system. In most cases the two strips are positioned opposite of each other, one on each side at the very end of its drainable tail. In other cases the two strips may be positioned side by side on one side and parallel with the bottom end. The strips may be constructed from a more or less rigid plastic or from a softer polymeric foam. The major advantage of such a pouch locking system is, that the 'tail clip' is integral, so it cannot be lost and it is as small and flexible as possible, which is a great advantage in comfort over the rigid plastic clips. The disadvantage of such a soft integral closure is, that sometimes it is difficult to open the pouch tail after rolling it out, in order to dispose of the stool.

It is often rather difficult to keep the fingers clean, when disposing of the stool or when cleaning the drainable pouch opening.

### Description of the invention

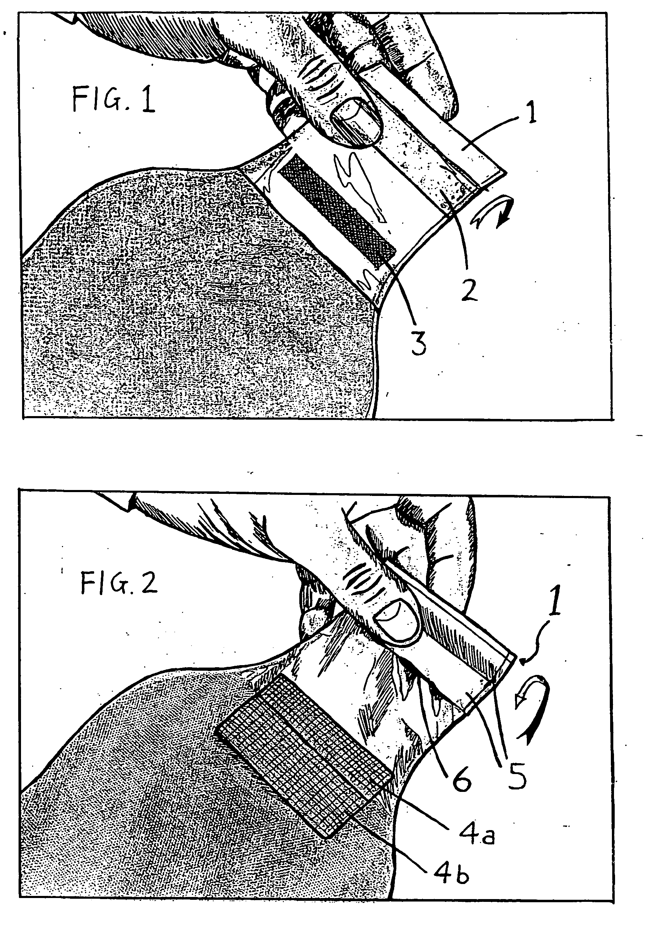

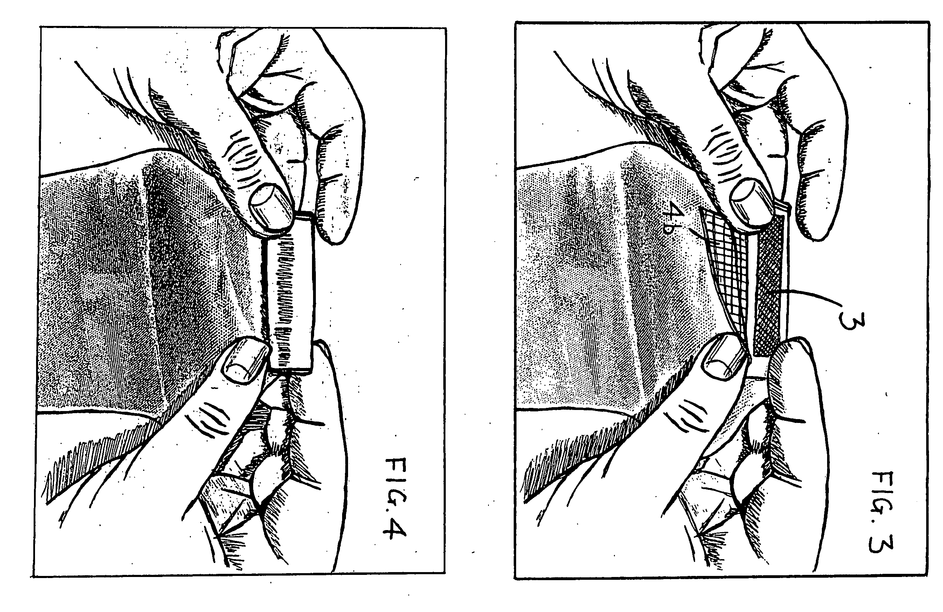

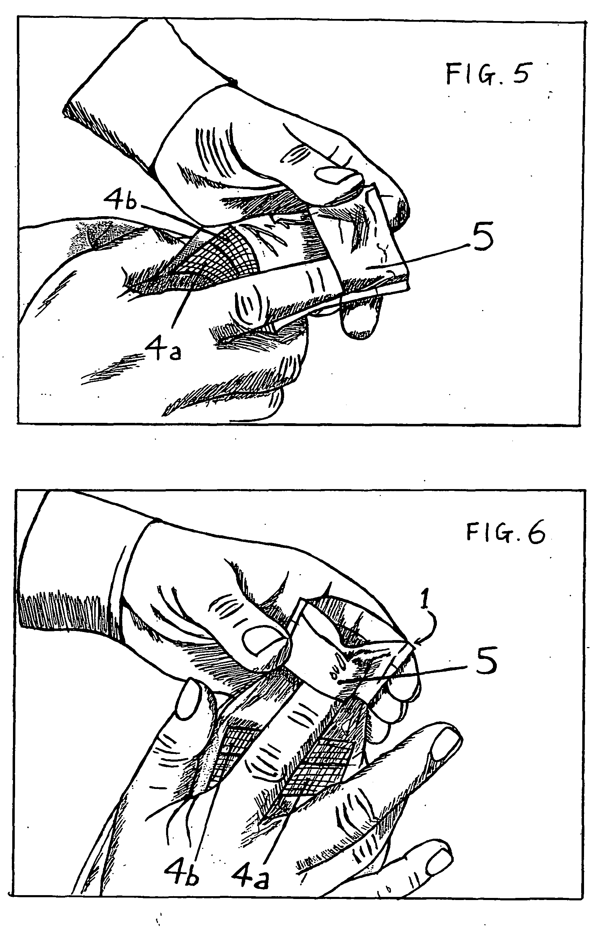

This invention comprises an integral, soft and flexible pouch tail closing system, to be used on drainable stoma pouches, applicable for both and 1-piece or 2-piece stoma systems, which type of pouches are widely in use by people with ileostomas. In order to create a relatively rigid, but yet flexible base to roll up the drainable pouch tail, its very end is provided with two parallel plastic strips (see Fig. 1 and 2). These two strips, each ca. 15 mm wide, are bonded side by side on the skin facing pouch end and are parallel with the bottom end of the pouch. The bottom side of the lowest strip is equal with the drainable end of the pouch tail. On the same (skin facing) side of the pouch tail, approximately one strip width higher positioned than the upper plastic strip, a small strip of a male velcro-like fastening material is bonded to the pouch film (see Fig. 1 and 2). However, this velcro-like material does not include hooks, but numerous, very small mushroom like elevations, with extremely thin circular hoods. These hoods are designed to grip into the special loops of a female type of velcro. This female type of velcro is bonded on a plastic flap, which is attached on the pouch film of the other side of the pouch, just at the point where the pouch tail widens and the actual pouch begins (see Fig. 2). This flap is semi-attached to the pouch film, outwardly exposing the female velcro surface. When folding up the pouch tail several times over the two plastic strips, until the female velcro flap is pressed over the velcro-like male strip on the opposite side of the pouch tail, the pouch end is securely locked (see Fig 3 and 4). Apart from this special type of velcro fastening material, used in this invention, the so far described integral pouch locking system is not new, but more or less in conformity with different other integral stoma pouch locking systems. The innovation and quintessence of this integral pouch locking system, are in the elegant way the drainable pouch can be opened and locked by means of a 'finger pocket'. On the same side of the pouch as the female velcro flap, on the very bottom of the pouch tail and opposite the two plastic strips, a relatively small pocket is formed over the full width of the outlet, which is closed on the bottom en open at the top. This pocket is designed as 'finger pocket', to accommodate one or two fingertips, which help to open the pouch closure in a very hygienic way (see Fig. 5 and 6).

### Description of the drawings

Fig. 1 shows the skin facing side of the pouch tail, with the two parallel plastic strips (1 and 2) bonded on the very end of the pouch tail, and the relatively small strip of male 'hook-less' velcro (3). Seen from this side, one starts folding the two strips together and continue folding up the pouch tail in clockwise direction.
Fig. 2 shows the opposite side of the pouch tail. This drawing shows the female velcro flap (4a and 4b) exposing its side covered with loops, whereby the back of its lowest part (4a) is bonded to the pouch film and the upper part (4b) is kept free to move. On the bottom of the pouch tail a finger pocket (5) has been formed, which is closed on three sides and can be opened at the top (6). Seen from this side one starts folding the two strips together and continue folding up the pouch tail in counter-clockwise direction.
Fig 3 shows the pouch tail folded up, almost in locking position, with the small strip of male 'hook-less' velcro (3) brought in opposition with the loops of the upper part of the female velcro flap (4b).
Fig. 4 shows the pouch closure totally folded up in locked position.
Fig. 5 shows the finger pocket (5) with a finger in position.
Fig. 6 shows the finger pocket (5) lifted up, with the two parallel plastic strips (1 and 2) somewhat bend together in their length direction, thus resulting in the opening of the pouch tail outlet.

## Claims

1. Drainable stoma pouch comprising a drainable pouch tail, the pouch tail comprising at least a first strip (1) of flexible material parallel with the bottom end of the pouch **characterised in that** the pouch tail further comprises a finger pocket (5) comprising an opening (6) pointing away from the pouch tail, the finger pocket being located on the pouch tail opposite to the first strip of flexible material

2. Drainable stoma pouch according to claim 1, further comprising a second strip (2) of flexible material parallel to the first strip provided on the same side of the pouch as the first strip.

3. Drainable stoma pouch according to claim 1, wherein the finger pocket is closed on three sides.

4. Drainable stoma pouch according to claim 1, wherein the flexible material is plastic.

5. Drainable stoma pouch according to claim 1, further comprising:
- a first strip (3) of Velcro-like material at the same side of the pouch as the first **strip of flexible material; and**
- a second strip (4a, 4b) of Velcro-like material provided on a side of the pouch **opposite to the side where the first strip is provided;**
the first strip and the second strip of Velcro-like material being located such that when the pouch tail is rolled up several times over the strip of flexible material, the pouch end can be securely locked by pressing the first strip of Velcro-like material over the second strip of Velcro-like material.

6. Drainable stoma pouch according to claim 5, wherein
- the first strip of Velcro-like material is of a male-type
- and the second strip of Velcro-like material is of a female-type

7. Drainable stoma pouch according to claim 5, wherein a part (4b) of the second strip of Velcro-like material parallel to the bottom end of the pouch and furthest away from the bottom end of the pouch is not attached to pouch.

8. Drainable stoma pouch according to claim 5, wherein the first strip of Velcro-like material is parallel to the first strip of flexible material.

## Patentansprüche

1. Entleerbarer Stomabeutel mit einem entleerbaren Beutelende, wobei das Beutelende zumindest einen ersten Streifen (1) aus einem flexiblen Material parallel zum unteren Ende des Beutels umfasst, **dadurch gekennzeichnet, dass** das Beutelende ferner eine Fingertasche (5) mit einer vom Beutelende weg weisenden Öffnung (6) umfasst, wobei die Fingertasche auf dem Beutelende gegenüber dem ersten Streifen aus flexiblem Material angeordnet ist.

2. Entleerbarer Stomabeutel nach Anspruch 1, der ferner einen zweiten Streifen (2) aus einem flexiblen Material parallel zum ersten Streifen umfasst, der auf derselben Beutelseite wie der erste Streifen angebracht ist.

3. Entleerbarer Stomabeutel nach Anspruch 1, worin die Fingertasche auf drei Seiten geschlossen ist.

4. Entleerbarer Stomabeutel nach Anspruch 1, worin das flexible Material Kunststoff ist.

5. Entleerbarer Stomabeutel nach Anspruch 1, der ferner Folgendes umfasst:
- einen ersten Streifen (3) aus einem klettbandartigen Material auf derselben Beutelseite wie der erste Streifen aus flexiblem Material; und
- einen zweiten Streifen (4a, 4b) aus einem klettbandartigen Material auf einer Beutelseite gegenüber der Seite, an welcher der erste Streifen angebracht ist;
wobei der erste Streifen und der zweite Streifen aus klettbandartigem Material so angeordnet sind, dass das Beutelende durch Festdrücken des ersten Streifens aus klettbandartigem Material über den zweiten Streifen aus klettbandartigem Material sicher fixiert werden kann, wenn das Beutelende mehrmals über dem Streifen aus flexiblem Material aufgerollt wird.

6. Entleerbarer Stomabeutel nach Anspruch 5, worin
- der erste Streifen aus klettbandartigem Material vom männlichen Typ ist
- und der zweite Streifen aus klettbandartigem Material vom weiblichen Typ is.

7. Entleerbarer Stomabeutel nach Anspruch 5, worin ein Teil (4b) des zweiten Streifens aus klettbandartigem Material, der parallel zum unteren Beutelende verläuft und vom unteren Beutelende am weitesten entfernt ist, nicht am Beutel befestigt ist.

8. Entleerbarer Stomabeutel nach Anspruch 5, worin der erste Streifen aus klettbandartigem Material parallel zum ersten Streifen aus flexiblem Material verläuft.

## Revendications

1. Poche stomale purgeable comprenant une extrémité de poche purgeable, l'extrémité de poche comprenant au moins une première bande (1) en matériau souple parallèle à l'extrémité de base de la poche, **caractérisée en ce que**
l'extrémité de poche comprend en outre une poche de doigt (5) dotée d'une ouverture (6) non tournée vers l'extrémité de poche et
**en ce que** la poche de doigt est située sur l'extrémité de poche située face à la première bande en matériau souple.

2. Poche stomale purgeable selon la revendication 1, comprenant en outre une deuxième bande (2) en matériau souple prévue sur le même côté de la poche que la première bande.

3. Poche stomale purgeable selon la revendication 1, dont la poche de doigt est fermée sur trois côtés.

4. Poche stomale purgeable selon la revendication 1, dont le matériau souple est une matière plastique.

5. Poche stomale purgeable selon la revendication 1, comprenant en outre :
- une première bande (3) en matériau de type Velcro sur le même côté de la poche que la première bande (1) en matériau souple,
- une deuxième bande (4a, 4b) en matériau de type Velcro sur le côté de la poche situé face au côté sur lequel la première bande est prévue,
la première bande et la deuxième bande en matériau de type Velcro étant disposées de telle sorte que lorsque l'extrémité de poche est enroulée plusieurs fois sur la bande en matériau souple, l'extrémité de poche peut être bloquée de manière sûre en repoussant la première bande en matériau de type Velcro contre la deuxième bande en matériau de type Velcro.

6. Poche stomale purgeable selon la revendication 5, dans laquelle :
- la première bande en matériau de type Velcro est de type mâle et
- la deuxième bande en matériau de type Velcro est de type femelle.

7. Poche stomale purgeable selon la revendication 5, dans laquelle une partie (4b) de la deuxième bande en matériau de type Velcro parallèle à l'extrémité de base de la poche et la plus éloignée de l'extrémité de base de la poche n'est pas reliée à la poche.

8. Poche stomale purgeable selon la revendication 5, dans laquelle la première bande en matériau de type Velcro est parallèle à la première bande en matériau souple.
